# EUROPEAN PATENT SPECIFICATION

(11) **EP 0 716 840 B1**
(45) Date of publication and mention of the grant of the patent: **08.05.2002**
(21) Application number: 95119558.5
(22) Date of filing: 12.12.1995
(51) Int. Cl.: A61F 2/44, A61F 2/46

(54) **Conically-shaped fusion cage**
Konischer Fusionskäfig
Cage de fusion conique

(30) Priority: 12.12.1994 US 354364
(43) Date of publication of application: 19.06.1996
(60) Divisional application: 01123254.3
(73) Proprietor: Surgical Dynamics, Inc., Norwalk, CT 06856 (US)
(72) Inventor: Pavlov, Paul W., Sint Maartenskliniek, NL-6522 JV Nijmegen (NL); Winslow, Charles J., Walnut Creek, CA 94595 (US); Jayne, Kirk, Alameda, CA 94501 (US); Klyce, Henry A., Piedmont, CA 94611 (US)
(74) Representative: Marsh, Roy David

(56) References cited:
- EP-A- 0 307 241
- EP-A- 0 551 187
- WO-A-87/07827
- WO-A-89/12431
- WO-A-91/06261
- WO-A-94/17759
- DE-A- 3 505 567
- DE-A- 4 302 397
- DE-C- 4 323 595
- FR-A- 2 350 824
- FR-A- 2 710 519

## Description

### BACKGROUND

### Field of the Invention

The present invention is directed to devices for facilitating the fusing of bone structures and more particularly the fusing together of adjacent vertebral bodies or bone structures.

### Background of the Invention

Technical literature and patent documents disclose a number of devices and methods for fusing bones together. One such device which has proven to be successful is disclosed in U.S. Patent 4,961,740, entitled "V-THREAD FUSION CAGE AND METHOD OF FUSING A BONE JOINT," which patent has been assigned the present assignee. The referenced patent discloses a fusion cage which is preferably cylindrical and has a thread formed as part of the external cylindrical surface. The fusion cage defines an internal cavity and apertures through the wall of the cage which communicate the external cylindrical surface with the internal cavity. The apertures are formed in the valleys of the thread. Normally two such cages are used to stabilized and fuse together adjacent vertebral bodies or bone structures.

In practice, using a posterior approach, a patient's vertebral bone structures are exposed and degenerate disk material located between the vertebral bone structures is removed. A threaded tap is used to tap a complementary thread in the upper and lower vertebral bone structures preparatory to the insertion of the above fusion cage. Once such tapping has been accomplished, using an introduction tool, the fusion cage is screwed into the space between the adjacent vertebral bone structures. The thread bites into the bone of the upper and lower vertebral bone structures, stabilizing the bone structures, and preventing the fusion cage from working out of this position due to patient movement. Generally two such fusion cages are applied using this technique. Once the two implants have been positioned, then bone growth inducing substances, such as bone chips, are packed into the internal cavity of the fusion cages. These bone growth inducing substances come into immediate contact with the bone from the vertebral bone structures which project into the internal cavity through the apertures. Such projection of bone is due to the fact that the apertures are formed in the valleys of the external thread of the fusion cage. Such immediate bone to bone contact between the vertebral bone structures and the bone pack within the fusion cages results in more rapid propagation of bone cells between the adjacent vertebral bone structures and thus a more rapid fusion of the adjacent vertebral bone structures.

It is to be understood that in the above method, bone growth inducing substances can be prepacked into the cages before the cages are implanted between the vertebral body structures.

Document WO-A-87/07827 discloses an implant for securing two adjacent vertebrae which comprise a body having a proximal end and a distal end wherein said proximal and distal ends define opposite ends of a frusto-cone. The implant is inserted between the vertebral bone structures by hammering after a predetermined amount of bone structure has been removed.

Further, document WO 94/17759 discloses an implant which is a hollow cylinder having male threads exposed on the exterior cylindrical surface for implanting between two vertebrae.

Furthermore, document DE 43 02 397 discloses an implant to be inserted between two vertebrae which is secured to the bone structure by a plate and two screws. Additionally, the implant comprises a helical rib on the outer surface.

### Summary of the invention

It is the object of the present invention to provide a device for facilitating the fusion of bone structures which gradually spreads apart vertebral bone structures in order to regain or enlarge the natural lordosis of the adjacent vertebral bone structures.

This problem is solved by a fusion cage as defined in claim 1.

In a preferred embodiment, the distal end further is rounded with for example a bull nose in order to facilitate the insertion of the cage body relative to one or more bone structures. The distal end could alternatively have a snub nose with or without a starter turn of a thread. The snub nose has a starter diameter that is smaller than the diameter of the distal end. The cage body according to the invention is frusto-conically-shaped. This shape is particularly advantageous due to the fact that the normal lordosis of the vertebral bone structures defines a wedged-shape space for a vertebral disk between, for example, lumbar vertebrae. Accordingly, the conically-shaped body cage can be sized and selected in order to maintain or enlarge upon the normal lordosis.

In a preferred embodiment of the present invention the cage body has at least one flute formed in the outer surface. Such flutes act as a relief much as the flute placed on self-tapping screws in order to facilitate the insertion of the fusion cage using a twisting motion between two vertebral bone structures.

The cage body has an outer surface and a thread formed as part of the outer surface. The thread aids the cage body in being inserted. As the cage is inserted, it gradually spreads apart the vertebral bone structures in order to regain or enlarge the natural lordosis of the adjacent vertebral bone structures. As with other embodiments of the present invention, flutes can be provided in the thread in order to allow for enhanced thread tapping by the cage and for a smoother insertion of the fusion cage between the vertebral bone structures. Preferably two or three flutes would be formed spaced about the fusion cage in order that one flute would be engaging with or adjacent to an upper vertebral bone structures with another flute being engaging with or adjacent to a lower vertebral bone structure. Such a relationship maintains alignment of the fusion cage and prevent wandering as the fusion cage is introduced between the two vertebral bone structures. Without two or more flutes, wandering might occur due to the fact that the thread is only substantially engaged with the vertebral bone structures and not with the disk material between the vertebral bone structures, which disk material does not provide support to the thread.

In a further aspect of the invention, any of the above embodiments can be provided with a plurality of apertures through the fusion cage and an internal cavity with the apertures communicating between the internal cavity and the external surface of the fusion cage. Bone growth inducing substances, such as bone chips, can be packed into the internal cavity either before the fusion cage is inserted or after the fusion cage has reached a final insertion position, or packed in both before and after. The bone chips come in contact with the vertebral bone structures through the apertures in order to facilitate fusion between the adjacent vertebral bone structures.

In another aspect of the invention which can be included in any of the above embodiments, the cage body can have a round or bull nose distal end with one or more flutes formed in the round or bull nose distal end in order to enhance the self-tapping nature of the fusion cage and to prevent the cage from wandering.

In yet another aspect of the invention, introduction tools allow the fusion cage to be accurately positioned between the vertebral bone structures. A preferred introduction tool allows for the cage to be implanted and thereafter allows an end cap of the cage to be conveniently removed, if desired, in order to place bone growth inducing substances in the cage.

It is to be understood that although the above-embodiments have been described with respect to the fusion of adjacent vertebral bodies or bone structures, that the present invention can be used (1) to fuse together a variety of bone structures, in addition (2) to being fused to one bone structure and used as, for example, a base for an implant or (3) to being used to reunite the pieces of a broken bone.

Other objects and advantages of the invention can be obtained through a review of the specification and the figures.

### Brief Description of the Figure

### Anterior Fusion Cage:

Figure 1 is a partially sectional side view of an embodiment of the anterior fusion cage of the invention.

Figure 2 depicts a left end (distal end) view of the fusion cage of Figure 1.

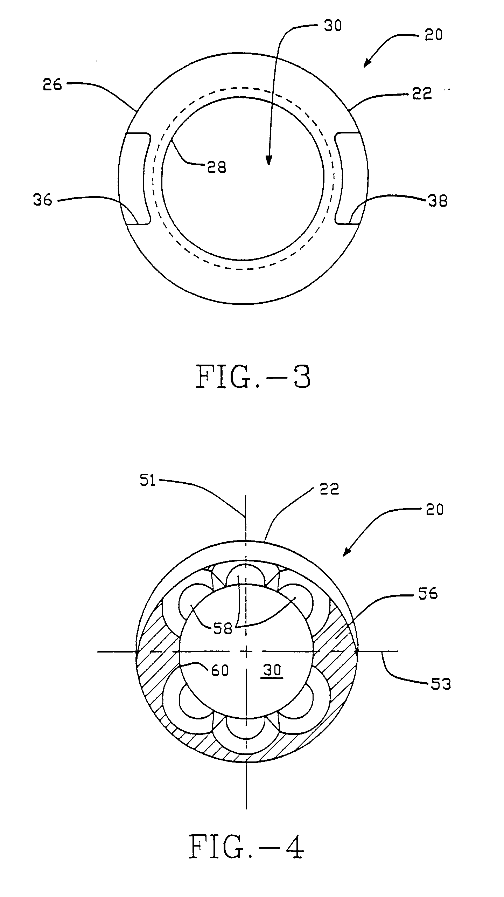

Figure 3 depicts a right end (proximal end) view of the fusion cage of Figure 1.

Figure 4 depicts a view through line 4-4 of the fusion cage of Figure 1.

Figure 5 depicts the fusion cage of Figure 1 in conjunction with an introduction tool.

Figure 6 depicts an alternative embodiment of the introduction tool.

Figures 7, 8, and 9 depict progressive stages in the method of inserting the anterior fusion cage between adjacent vertebral bone structures.

Figure 10 depicts a side view of an alternative embodiment of the anterior fusion cage of the invention.

Figure 11 depicts the left end (distal end) view of the fusion cage of Figure 10.

Figure 12 depicts the right end (proximal end) view of the fusion cage of Figure 10.

Figure 13 depicts a side view of yet another embodiment of the anterior fusion cage of the present invention.

Figure 14 depicts a left distal end (distal end) view of the fusion cage of the invention of Figure 13.

Figure 15 depicts a right end (proximal end) view of the fusion cage of the invention of Figure 13.

Figure 16 depicts a sectional view taken through line 16-16 of Figure 13.

### Posterior Fusion Cage:

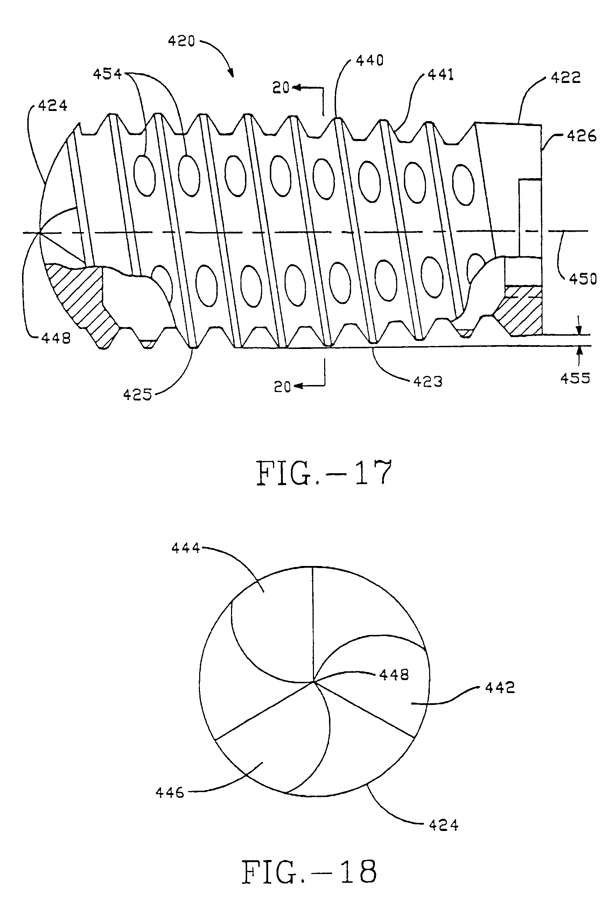

The posterior fusion cages depicted in Figs. 17-26, 27 and 29 do not represent the invention.

Figure 17 is a partially sectional side view of an embodiment of the posterior fusion cage.

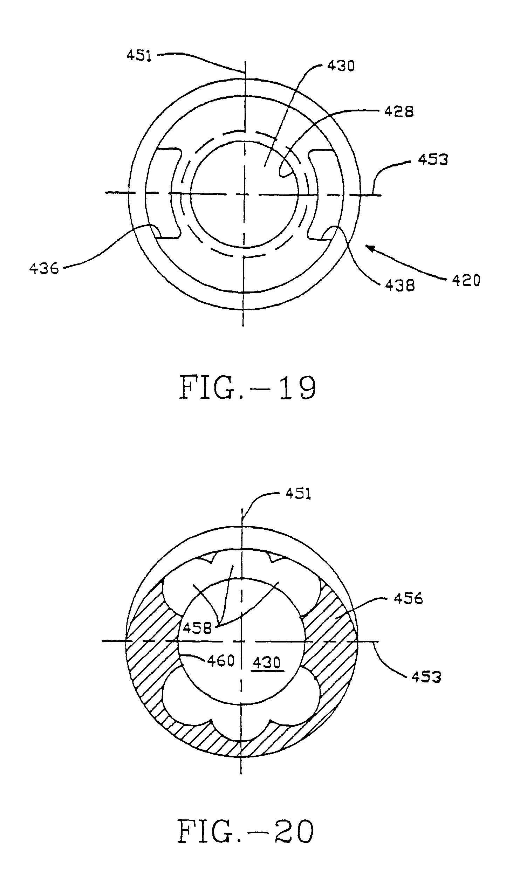

Figure 18 depicts a left end (distal end) view of the fusion cage of Figure 17.

Figure 19 depicts a right end (proximal end) view of the fusion cage of Figure 17.

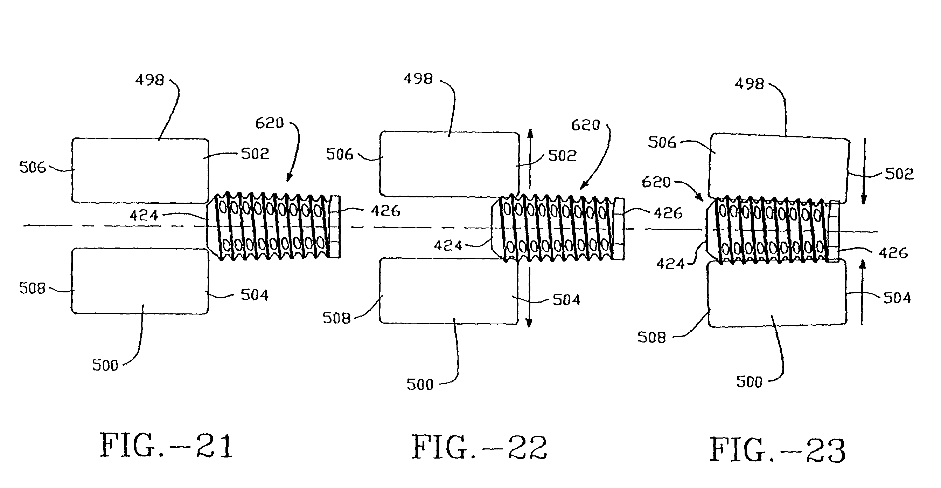

Figure 20 depicts a view through line 20-20 of the fusion cage of Figure 17.

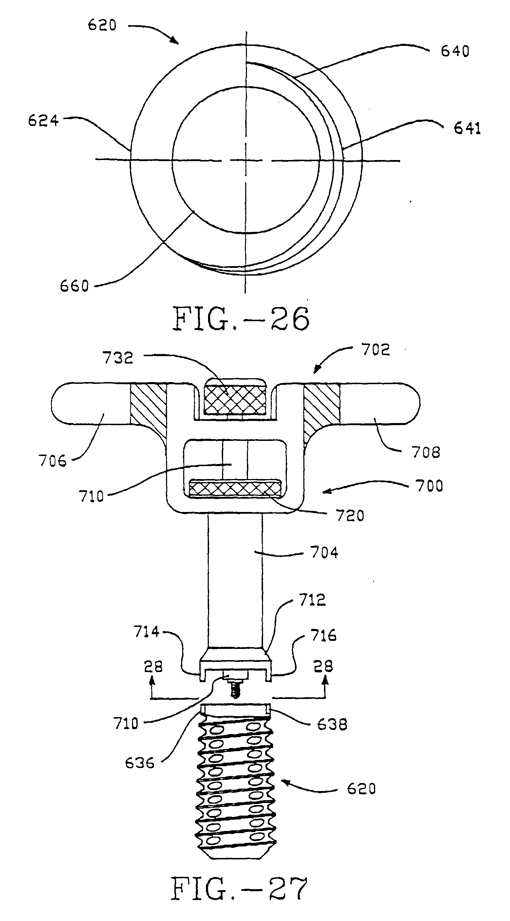

Figures 21, 22, and 23 depict progressive stages in the method of inserting the posterior fusion cage between adjacent vertebral bone structures using the cage depicted in Figure 25.

Figure 24 depicts a side view of an alternative embodiment of the posterior fusion cage.

Figure 25 depicts a side view of another embodiment of the posterior fusion cage.

Figure 26 depicts a left end (distal end) view of the embodiment of the fusion cage of Figure 25.

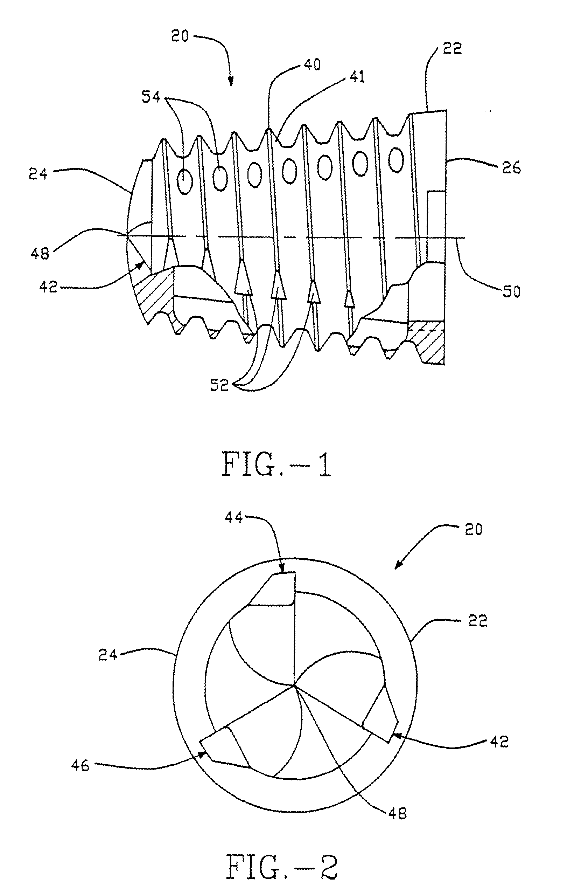

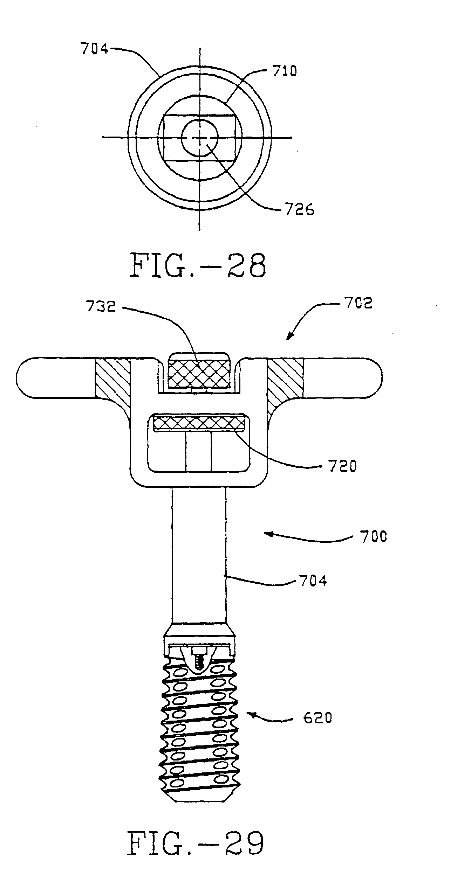

Figure 27 depicts the fusion cage of Figure 25 in conjunction with a new preferred insertion tool that can preferably be used with the anterior fusion cages of Figure 1, 10 and 13, and with the posterior fusion cages of Figure 17 and 25.

Figure 28 depicts an end view of the insertion tool of Figure 27 along line 28-28.

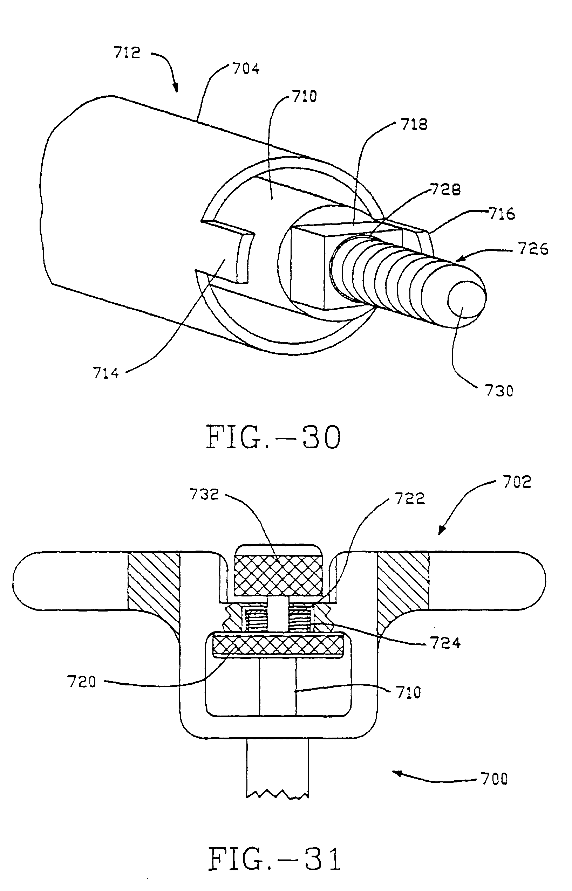

Figure 29 depicts a partially broken away view of the fusion cage and the insertion tool of Figure 27 connected together.

Figure 30 depicts a perspective view of the end of the insertion tool depicted in Figure 28.

Figure 31 depicts a partially sectional view of the handle of the insertion tool of Figure 27.

### Detailed Description of the Preferred Embodiment

### Anterior Fusion Cage:

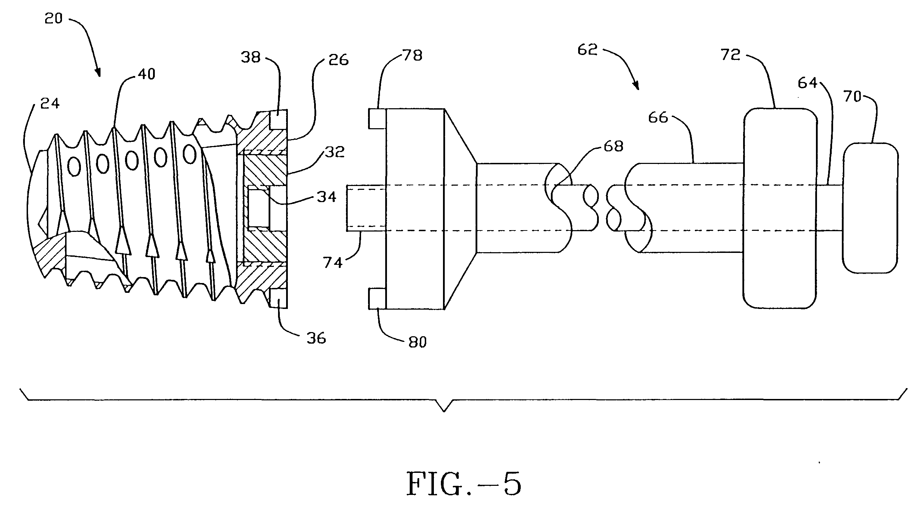

With respect to the figures in a particular Figure 1, a side view of the preferred embodiment of the fusion cage 20 is depicted. Fusion cage 20 includes a fusion cage body 22 which is provided in the shape of a cone. Fusion cage 20 includes a distal end 24 and a proximal end 26. The distal end 24 in a preferred embodiment is rounded or bull nosed in order to facilitate the insertion of the fusion cage 20 relative to one or more bone structures. The proximal end 26 includes an opening 28 which communicates with an internal cavity 30 defined by the fusion cage 20. The opening 28 in a preferred embodiment is threaded so that it can receive an end cap or plug 32 (Figure 5). End cap 32 is used to close off the proximal end 26 and retain bone growth inducing substances packed therein as described herein-below. As can be seen in Figure 5, end cap 32 includes a threaded bore 34 which is designed to receive an insertion tool. The threaded bore 34 has an initial unthreaded, square or hex-shaped section 35 which can be used with a socket wrench to tightly position end cap 32 in opening 28 and which can be engaged by a preferred insertion tool of Figure 27. Further the unthreaded portion of bore 34 could equally be cylindrical with an irregularity to allow for mating with an insertion tool, as well as having a variety of other shapes. The proximal end 26 further define first and second peripheral indentations 36, 38. These peripheral indentations 36, 38 receive tangs from an insertion tool as described hereinbelow for facilitating the insertion of the fusion cage 20.

A thread 40 is defined as part of the outer cylindrical surface 41 of the body 22. It is to be understood that the thread can be replaced with a plurality of discrete threads.

The rounded distal end 24, and at least some of the turns of thread 40 defined flutes or relief grooves 42, 44, and 46. (Figures 1, 2.) In a preferred embodiment, flutes 42, 44, and 46 meet at a central point 48 of the distal end 24 on the longitudal axis 50 of the fusion cage 20. In other embodiments the flutes can be smaller and not extend all the way to the central point 48 on the longitude axis 50. Still in other embodiments, the flutes can be eliminated from the distal end 24 and such embodiments are still within the scope of the invention.

The flutes extend from the distal end 24 toward the proximal end 26 as shown in Figure 1 with respect to flute 42. These flutes are defined by the sections 52 which are removed from the thread. In a preferred embodiment, the flutes become narrower as they approach the proximal end 26 due to the fact that thread relief for purposes of self-tapping becomes less important as the cage reaches a final resting position. As shown in other embodiments, the flutes can be deeper and extend from the distal end completely to the proximal end. Still further in other embodiments the flutes can be confined to the first several turns of the thread adjacent to the distal end and/or to just the distal end.

As can be seen in Figures 1, 4, a plurality of apertures 54 are provided through wall 56 of the fusion cage 20. In a preferred embodiment, these apertures 54 are formed by broaching grooves 58 in the internal surface 60 of the internal cavity 30. The effect of such broaching is to remove material from the valleys between the turns of the thread 40, thus defining the aperture 54. The advantages of such an arrangement are taught by the above-referenced U.S. Patent No. 4,961,740, and allows for immediate bone to bone contact between the vertebral bodies or bone structures and the bone packed within the internal cavity 30 of the fusion cage 20.

The apertures 54 in a preferred embodiment increase in size from smaller apertures closer to the distal end 24 to a larger aperture closer to the proximal end 26. This increase in size allows for more bone to bone contact. Alternatively in the embodiment as shown in Figure 1, all the apertures are of the same size.

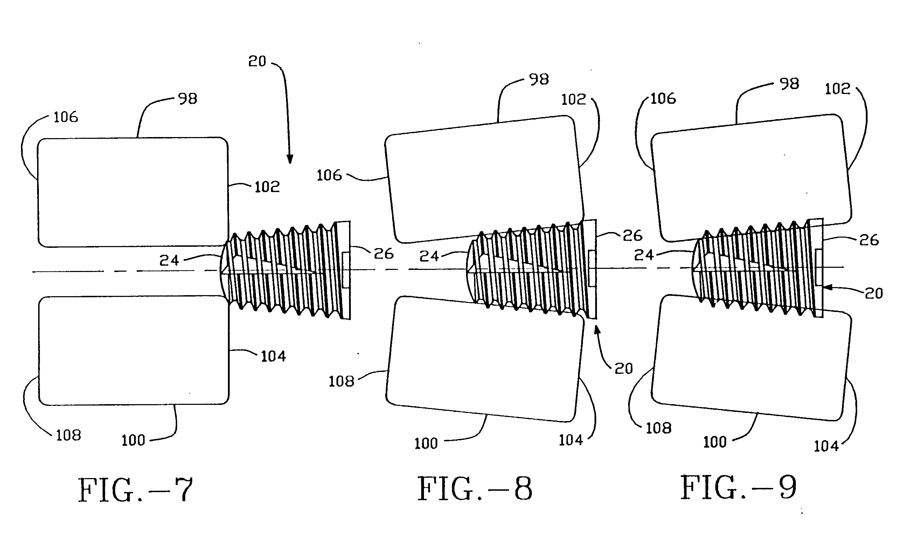

As can be seen in Figure 4, the apertures are clustered about a transverse axis 51, both at the upper and lower end of the axis. This is so that in position, the apertures come into contact with the upper and lower vertebral bone structures (Figure 9) to encourage bone growth through the fusion cage from the vertebral bone structures. The lateral section of the fusion cage found along the other transverse access 53 do not have apertures in order to prevent growth of disk material which might interfere with the bone fusing process.

A preferred embodiment of the conically-shaped fusion cage 20 includes a fusion cage which is 23 millimeters in length having a distal end 24 with a diameter of 14 millimeters and a proximal end 26 with a diameter of 18 millimeters. The cage body is a right circular cone. The thread has a pitch of 30° and there are ten turns per 2.54 cm (inch) with a thread depth of 0.135 cm (.053 inches). Further the cage is made of a titanium metal or alloy such as Ti64. Preferably this and the other disclosed fusion cages disclosed are machined. However, the processes such as molding, casting, or sintering can be used to accomplished formation of the fusion cages.

The cage is inserted between vertebral bodies using an insertion tool 62 (Figure 5). Insertion tool 62 includes an inner handle 64 and an outer handle 66. The outer handle includes a bore 68 for receiving the inner handle 64. Handles 64, 66 include knobs 70, 72 respectively. The distal end of inner handle 64 defines a threaded shaft 74, having a reverse thread to facilitate easy removal, and the distal end of handle 66 define a cylindrical disk 76 which has first and second tangs 78, 80, projecting from the peripheral edge of the cylindrical disk 76. These tangs 78, 80 are designed to mate with the peripheral indentation 36, 38 of the fusion cage 20. For purposes of inserting the fusion cage between the vertebral bodies, the end cap 32 is inserted into the fusion cage 20 as shown in Figure 5. Then the threaded shaft 74 of the inner handle is introduced into the threaded bore 34 of the end cap 32. After this is accomplished, the outer handle 66 is slid over the inner handle 64 and the tangs 78, 80 are positioned into engagement with the indentations 36, 38. In this arrangement, the fusion cage 20 can be anteriorly inserted into the space between the vertebral body structure using the insertion tool 62.

An alternative embodiment of the insertion tool is shown in Figure 6. In this figure, insertion tool 82 includes a handle 84 with a knob 86. At the end of the insertion tool 82 distal from the knob 86 is a cylindrical disk 88 which has first and second tangs 90, 92, which have the same function as the above tangs 78, 80. Extending from the center of the cylindrical disk 88 along the centerline of the insertion tool 82 is a shaft 94 which has a ball detent 96. For use with insertion tool 82, the threaded bore 34 of the end cap 32 would be replaced with a bore having a lip which could engage with the ball detent 96 of the insertion tool 82.

It is to be understood that the insertion tool depicted in Figure 27 and described below is preferable to the above described insertion tools for both the anterior fusion cages and the below described posterior fusion cages.

The method for inserting the fusion cage 20 of Figure 1 using an anterior approach and procedure to the vertebral bodies is as follows. It is to be understood that although the focus of this discussion is on a laparoscopic procedure, that the anterior approach and procedure can also include a more invasive procedure where a long incision is made in the abdomen wall.

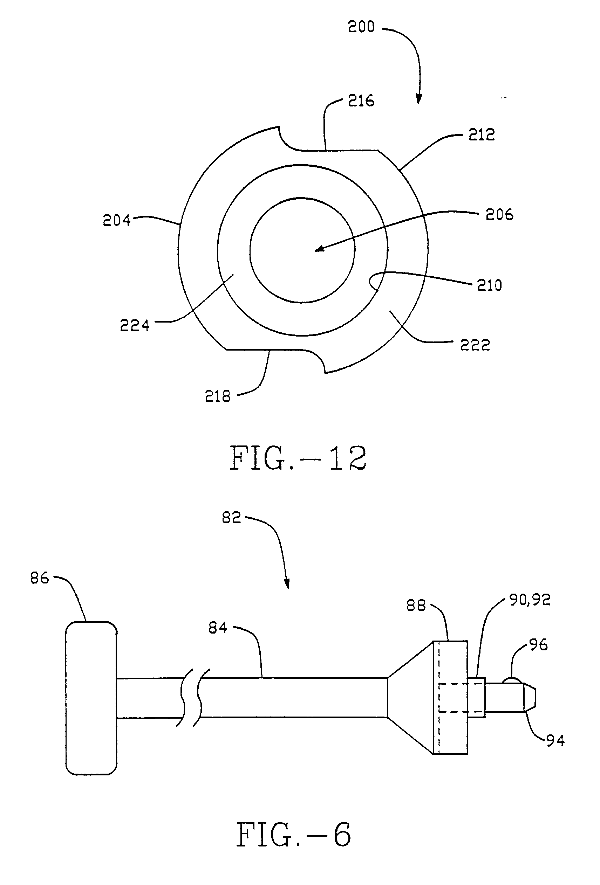

With an anterior approach, using an introduction set such as described by way of example only, in U.S. Patent 4,863,430, entitled "INTRODUCTION SET WITH FLEXIBLE TROCAR WITH CURVED CANNULA, " but however with larger diameter instruments, an amount of disk material is removed between the two vertebral bodies or bone structures which are to be fused together. This procedure is accomplished through a cannula position adjacent to the vertebral bone structures. With the same or a larger diameter cannula, the fusion cage 20 can be introduced adjacent to the vertebral bone structures. In a first procedure, the fusion cage is packed with bone growth substances and the end cap 32 is affixed to the fusion cage 20. Insertion tool 62 is then secured to the fusion cage 20 and the fusion cage is guided through the cannula to a location adjacent to the upper and lower vertebral body such as presented schematically in Figures 7, 8, 9, by upper body 98 and lower body 100. In the initial position as shown in Figure 7, the fusion cage 20 is adjacent to the anterior surfaces 102, 104 of the vertebral bodies 98, 100. As the introduction tool is turned, the thread 40 of the fusion cage 20 bites into the vertebral bodies 98, 100. Further turning of the introduction tool causes the fusion cage to move through the position shown in Figure 8 to the final resting position shown in Figure 9, where the distal end 24 is moved adjacent to the posterior sections 106, 108 of the vertebral bone structures 98, 100. As this occurs, the fusion cage 20 increases the lordosis or spacing between the vertebral bodies, basically distracting the vertebral bodies and causing the vertebral bodies to pivot about the posterior sections 106, 108, with such posterior sections acting like a hinge. It is noted that most of the distraction occurs adjacent to the anterior sections, but that distractions also occur at the posterior sections where the hinged effect is exhibited. Preferably, the lordosis is increased over the normal lordosis in order to stabilize the vertebral bone structures prior to fusion occurring. Stabilization occurs due to the fact that increased lordosis places additional stress on the anterior longitudinal ligaments which are part of the anatomy holding the vertebral bodies in place.

Once the fusion cage 20 is appropriately positioned, the handle 64 of the insertion tool 62 is unscrewed from the cap 32 and the fusion handle 62 is pulled away from the fusion cage.

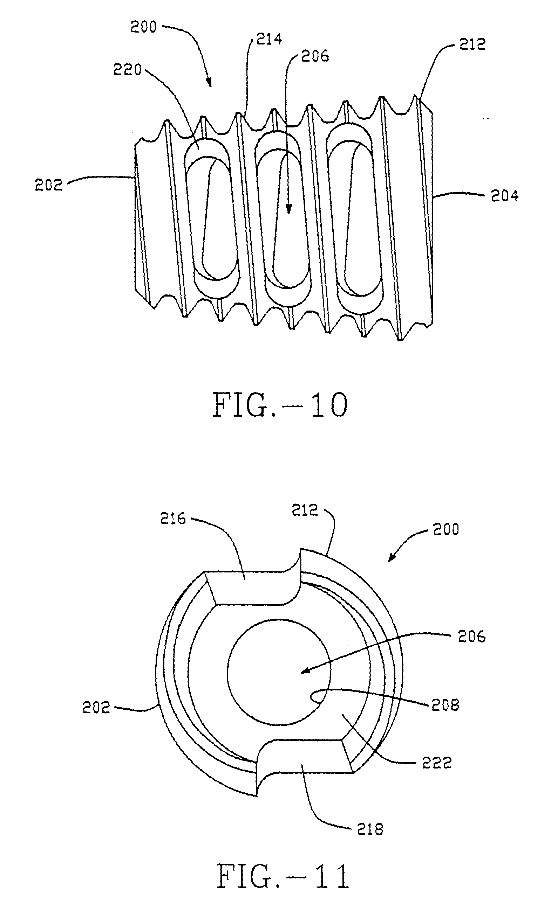

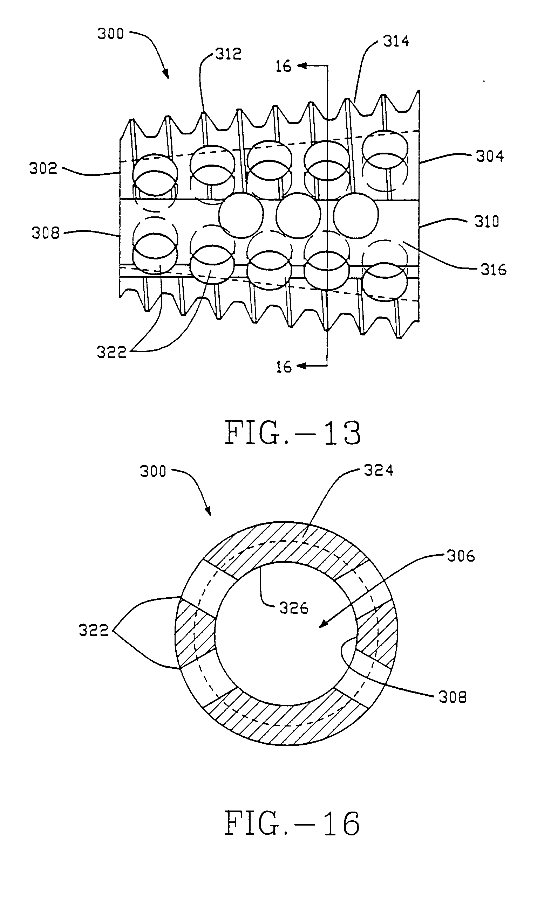

An alternative embodiment of a fusion cage 200 is shown in Figures 10, 11, and 12. Fusion cage 200 includes a distal end 202 and an a proximal end 204. Fusion cage 200 includes an internal cavity 206. End caps not shown can be used to close the ports 208, 210 of distal and proximal ends 202, 204. A thread 212 is defined on the external conical surface 214 of the fusion cage 200. Defined by the thread 212 are first and second flutes 216, 218, which in this embodiment extend from the distal end 202 to the proximal end 204. These flutes provide thread relief allowing the fusion cage 200 to be self-tapping.

The fusion cage 200 includes a plurality of elongated apertures 220 which are formed through the side walls of a fusion cage 200. The elongated apertures 202 are formed in such a way that the internal conical surface 214 is spaced away from the internal surface 224 of the internal cavity 206 by the thickness of the sidewall 222.

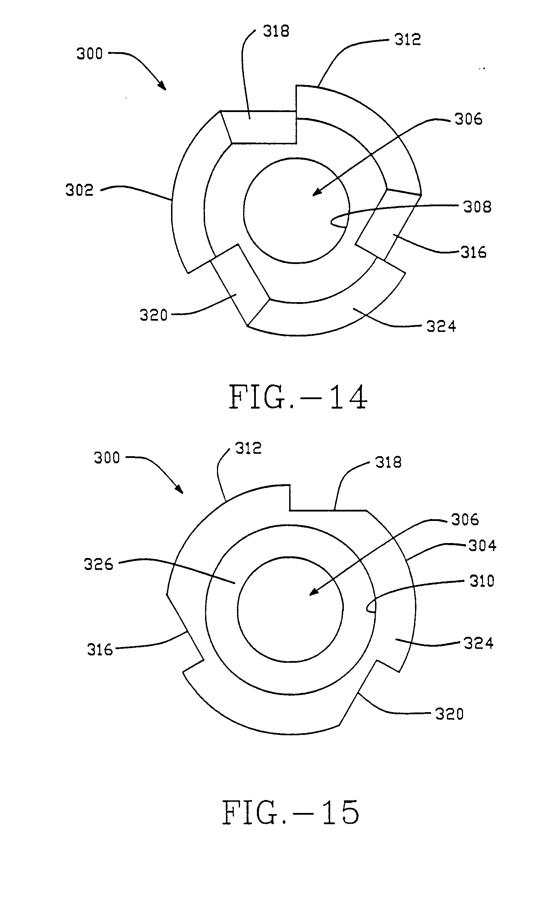

A further embodiment of the invention is shown in Figures 13, 14, 15 and 16. In Figure 16 the fusion cage 300 has distal and proximal ends 302 and 304 respectively. The fusion cage 300 defines an internal cavity 306, and ports 308 and 310 defined through the distal and proximal ends 302 and 304 respectfully. A thread 312 is defined as part of the external conical surface 314 of the fusion cage 200. First, second and third flutes 316, 318, and 320, are defined in the thread 312 from the distal end 302 to the proximal end 304. These flutes give the fusion cage 300 an enhanced self-tapping advantage. These flutes are equally spaced about the fusion cage 300 in a manner similar to the flutes of the fusion cage embodiment 20 in Figure 1.

A plurality of aperture 322 is provided through the external conical surface 314 of the fusion cage 300 and through the side wall 324 opening into the internal cavity 306. Accordingly, at the location of the aperture 322 the external surface 314 is held away from the internal surface 326 by the thickness of the side wall 324.

### Posterior Fusion Cage:

With respect to the figures in a particular Figure 17, a side view of the preferred embodiment of the posterior fusion cage 420 is depicted. Fusion cage 420 includes a fusion cage body 422 which in this preferred embodiment is provided with a conically-shaped portion 423 and a cylindrically-shaped portion 425. It is to be understood that alternatively the entire body 422 can be conically-shaped. Further, as appropriate the shape of the cage body 422 can be more complex with various conical and/or cylindrical configurations. Fusion cage 420 includes a distal end 424 and a proximal end 426. The distal end 424 in a preferred embodiment is rounded or bull nosed in order to facilitate the insertion of the fusion cage 420 relative to one or more bone structures. The proximal end 426 includes an opening 428 (Figure 19) which communicates with an internal cavity 430 (Figure 20) defined by the fusion cage 420. The opening 428 in a preferred embodiment is threaded so that it can receive an end cap or plug such as 32 of the embodiment in Figure 5. End cap is used to close off the proximal end 426 and retain bone growth inducing substances, such as bone chips, packed therein as described herein-below. As can be seen in the embodiment of Figure 5, end cap 32 includes a threaded bore 34 which is designed to receive an insertion tool. The threaded bore 34 has an initial unthreaded, square or hex-shaped portion 35 which can be used with a socket wrench to tightly position end cap 32 in opening 428 and which can also be engaged by the insertion tool of Figure 27 described below. Portion 35 can be otherwise shaped as described above.

The proximal end 426 of the embodiment of Figure 19 further define first and second peripheral indentations 436, 438 which are centered about transverse axis 453. These peripheral indentations 436, 438 receive tangs from an insertion tool as described below for facilitating the insertion of the fusion cage 420. These identifications are also used to line up the cage 420 for proper insertion between the vertebral bodies as discussed below.

A thread 440 is defined as part of the outer cylindrical surface 441 of the body 422. It is to be understood that the thread can be replaced with a plurality of interrupted or discrete threads or a plurality of projections, ridges, protrusions, barbs, or spurs.

The rounded distal end 424, and at least some of the turns of thread 440 can in a preferred embodiment can define flutes or relief grooves 442, 444, and 446 (Figures 24, 25). It is to be understood that in alternative embodiments the flutes can be eliminated from the distal end 424 and the thread 440, since for example, the bore for the insertion of the fusion cage 420 between the vertebral bodies can be pre-tapped. Still in alternative embodiment, the flutes on the distal end can remain to assist in the insertion of the cage 420 between the vertebral bodies. In a preferred embodiment, flutes 442, 444, and 446 meet at a central point 448 of the distal end 424 on the longitudal axis 450 of the fusion cage 420. In other embodiments the flutes can be smaller and not extend all the way to the central point 448 on the longitude axis 450. Still as indicated above in other embodiments, the flutes can be eliminated from the distal end 424 and the thread 440.

The flutes can extend from the distal end 424 toward the proximal end 426 as shown in the alternative embodiment in Figure 24 with respect to flute 542. These flutes are defined by the sections 552 which are removed from the thread. In this embodiment, the flutes become narrower as they approach the proximal end 526 due to the fact that thread relief for purposes of self-tapping becomes less important as the cage reaches a final resting position. As shown in other embodiments, the flutes can be deeper and extend from the distal end completely to the proximal end. Still further in other embodiments the flutes can be confined to the first several turns of the thread adjacent to the distal end and/or to just the distal end.

With respect to Figures 17, 20, a plurality of apertures 454 are provided through wall 456 of the fusion cage 420. In a preferred embodiment, these apertures 454 are formed by broaching grooves 458 in the internal surface 460 of the internal cavity 430. The effect of such broaching is to remove material from the valleys between the turns of the thread 440, thus defining the aperture 454. The advantages of such an arrangement are taught by the above-referenced U.S. Patent No. 4,961,740, and allows for immediate bone to bone contact between the vertebral bodies or bone structures and the bone packed within the internal cavity 430 of the fusion cage 420.

The apertures 454 in a preferred embodiment increase in size from smaller apertures closer to the proximal end 426 to a larger aperture closer to the distal end 424. This increase in size allows for more bone to bone contact. Alternatively in the embodiment as shown in Figure 17, all the apertures are of the same size.

As can be seen in Figure 20, the apertures are clustered about a transverse axis 451, both at the upper and lower end of the axis. This is so that in position, the apertures come into contact with the upper and lower vertebral bone structures (Figure 23) to encourage bone growth through the fusion cage from the vertebral bone structures. The lateral sections of the fusion cage found along the other transverse axis 453 do not have apertures in order to prevent growth of disk material which might interfere with the bone fusing process. As can be seen viewing both Figures 19 and 20 together, the indentation 436 and 438 are centered on the axis 453 with the aperture 454 centered on axis 451. Axis 451 is preferably perpendicular to axis 453. The insertion tool has tangs that are inserted in indentation 436 and 438. Accordingly, the position of the insertion tool defines the position of the apertures 454 in that upon insertion the apertures 454 can be put in contact with the upper and lower vertebral bodies to allow bone ingrowth and prevent lateral ingrowth of disk material.

A preferred embodiment of the conically-shaped fusion cage 420 includes a fusion cage which is 28 millimeters in length having a distal end 424 with a diameter of 14 millimeters and a proximal end 426 with a diameter of 16 millimeters. The cage body is a right cylinder from the distal end 424 extending toward the proximal end 426 for four turns of thread 440. Then the cage 420 becomes a right cone from the remaining five turns of thread 440 until thread 440 terminates at proximal end 426. This conically-shaped portion is defined by relief 455 of 3.2°.. The thread has a pitch of 30° and there are ten turns per 2.54 cm (inch) with a thread depth of 0,135 cm (.053 inches). Further the cage is made of a titanium metal or alloy such as Ti64. Preferably this and the other disclosed fusion cages disclosed are machined. However, the processes such as molding, casting or sintering can be used to accomplished formation of the fusion cages.

The cage is inserted between vertebral bodies using a preferred insertion tool 700 shown in Figure 27. Insertion tool 700 includes a handle 702 with an outer shaft 704 extending therefrom. The handle 702 includes first and second wings 706, 708 which make the handle easier to grab. The outer shank 704 is hollow and disposed within the outer shaft is an intermediate shaft 710 which can be seen extending from the cage engaging in 712 of the shaft 704. The cage engaging end 712 includes first and second tangs 714, 716 which can be inserted in the indentation of the cage such as for example indentations 436, 438, as shown in Figure 19, and indentations 636 and 638 shown in Figure 27. The end of shaft 710 includes a square-shaped drive 718 which engages the square-shaped unthreaded portion of the otherwise threaded bore such as bore 34 of an end cap such as end cap 32 as shown in Figure 5. This same end cap can be used in the end of fusion cage 620. Alternatively, the square drive can be hexagonal shape with the unthreaded portion of the bore 34 being hexagonal shaped to provide the necessary mating arrangement. Other mating shapes can also be used. A first knurled knob 720 is secured to immediate shaft 710 in order to provide a mechanism for rotating intermediate shaft 710 inside of outer shank 704. As can be seen in Figure 31, the intermediate shank 710 is spring biased relative to the handle 702 with a spring 722. Spring 722 is imbedded in a bore 724 of handle 702. In Figure 31, the first knurled knob 720 and the shank 710 are pulled back and thus compress the spring 722. In Figure 27, the first knurled knob 720 is released and the spring (not shown) is uncompressed.

An inner shaft 726 is located within a bore 728 of the intermediate shaft 710. The inner shaft 726 ends in a threaded portion 730 (Figure 30). The other end of inner shaft 726 is secured to the second knurled knob 732. Inner shaft 726 is free to rotate, through the use of the second knurled knob 72, within the bore 728 of the intermediate shaft 710. In addition the inner shaft 728 has limited freedom of motion along the longitudinal axis of the inner shaft 726.

The operation of the insertion tool 700 is as follows. With the insertion tool 700 not secured to a fusion cage, the insertion tool is as depicted in Figure 27 with the threaded portion 730 being either received entirely within bore 728 or extending a minimal amount out of bore 728. With the end cap secured in the fusion cage, the exposed square drive 718 is mated with the square portion of the bore in the end cap. The tangs 712, 714 are aligned with the indentations 636 and 638 and the tool is pushed in so that the tang 712, 714 are received in the indentations 636, 638. As this occurs, the knurled knob 730 moves up to the position as shown in Figure 29 and 31, compressing the spring. After this occurs, the second knurled knob 732 can be turned clockwise in order to engage the threaded portion 730 of the inner shaft 726 with the threaded portion of the bore of the end cap. This draws the fusion cage securely to the insertion tool 700 as shown in Figure 29. In this position, the cage is ready for insertion between the vertebral bodies. The handle 702 is then used to screw the cage between the vertebral bodies into the final resting position. Once the cage is in the final resting position, second knurled knob 732 is turned counter-clockwise in order to back the threaded 730 out of the threaded portion of the bore of the end cap. As this occurs, the spring 722 causes the square drive 718 to push against the end cap maintaining the end cap in its position relative to the fusion cage until the threaded portion 730 disengages itself from the threaded portion of the end cap, and the insertion tool 700 is disengaged from the fusion cage and can be removed. Thus the square drive, which is spring loaded, prevents the end cap on the cage from screwing out when the insertion tool is removed from the cage.

Should it be desired to move the end cap with the fusion cage in place, the square drive 718 can be inserted into the square portion of the threaded bore. The threaded portion 730 of the inner shaft 726 can then be screwed into engagement with the threaded portion of the threaded bore of the end cap, preferably with the tangs unaligned with the indentations. The first knurled knob 720 can then be turned in order to back the cap out of the fusion cage. A reverse of this operation can be used to insert the end cap into the fusion cage after additional bone growth inducing substances are packed into the fusion cage.

The method for inserting the fusion cage 420 of Figure 17 using a posterior approach and procedure to the vertebral bodies is as follows. Both a minimally invasive procedure and a more invasive procedure where a long incision is made in the back can be used.

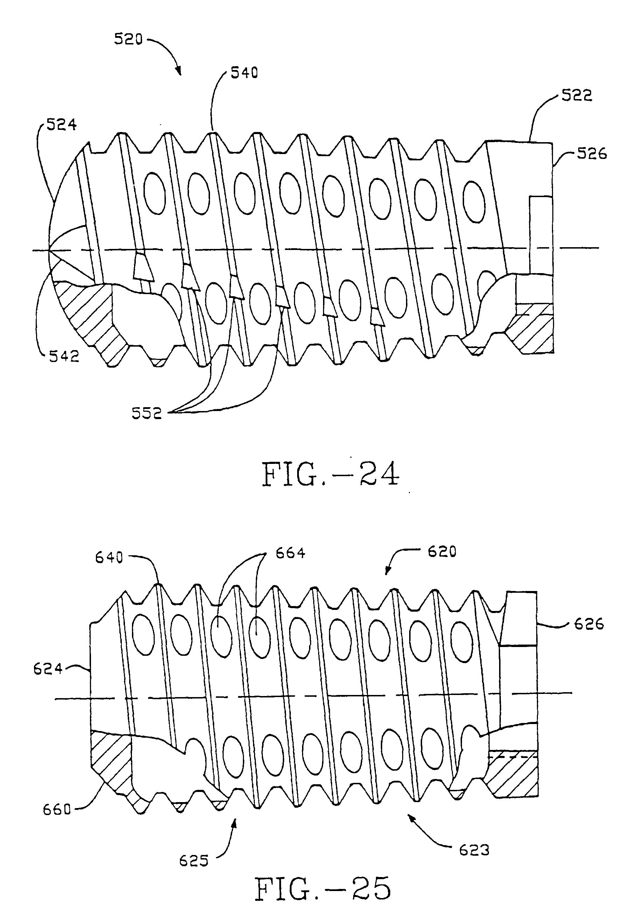

With a posterior approach, using an introduction set such as described by way of example only, in U.S. Patent 4,863,430, entitled "INTRODUCTION SET WITH FLEXIBLE TROCAR WITH CURVED CANNULA," but however with larger diameter instruments, an amount of disk material is removed between the two vertebral bodies or bone structures which are to be fused together. This procedure is accomplished through a cannula position adjacent to the vertebral bone structures. Then if required a thread is tapped in the upper and lower vertebral bodies. With the same or a larger diameter cannula, the fusion cage 420, or alternatively the preferred fusion cage 620 of Figure 25, can be introduced adjacent to the vertebral bone structures. In a first procedure, the fusion cage is packed with bone growth substances and the end cap is affixed to the fusion cage 620. Insertion tool 700 is then secured to the fusion cage 620 and the fusion cage is guided through the cannula to a location adjacent to the upper and lower vertebral body such as presented schematically in Figures 21, 22, 23, by upper body 498 and lower body 500. In the initial position as shown in Figure 21, the fusion cage 620 is adjacent to the posterior sections 502, 504 of the vertebral bodies 498, 500. As the introduction tool is turned, the thread 640 (Figure 25) of the fusion cage 620 bites into the vertebral bodies 498, 500. Further turning of the introduction tool causes the fusion cage to move through the position shown in Figure 22 to the final resting position shown in Figure 23, where the distal end 624 is moved adjacent to the anterior sections 506, 508 of the vertebral bone structures 498, 500. As this occurs, the fusion cage 620 increases the lordosis or spacing between the vertebral bodies, basically distracting the vertebral bodies. It is noted that most of the distraction occurs adjacent to the anterior sections, but that distraction also occur at the posterior sections. Preferably, the lordosis is increased over the normal lordosis in order to stabilize the vertebral bone structures prior to fusion occurring. Stabilization occurs due to the fact that increased lordosis places additional stress on the anterior longitudinal ligaments which are part of the anatomy holding the vertebral bodies in place.

Once the fusion cage 620 is appropriately positioned, the insertion tool 700 is unscrewed from the cap and the insertion tool 700 is pulled away from the fusion cage.

It is to be understood that the cage can be implanted without the use of a cannula by making a larger incision in the back. With this arrangement the bone chips would more often be packed into the cage after the cage reaches its final position and then an end cap would be secured t the cage. In the final position apertures 454 or 654 (embodiment of Figure 25) would be positioned adjacent vertebral bodies 498 and 500. No matter which procedure is used to insert the cage 420 or 620, it is advantageous to provide a bore between the vertebral bodies that is less than the diameter of the distal end 424. Thus, for a cage 420 or a cage 620 with a distal end having an 18 diameter, the bore would be 14 millimeters. Inserting the cage 420 or the cage 620 would cause the vertebral bodies to be distracted (Figure 22) and then rock back (Figure 23) onto the conically-shaped portion of the fusion cage 420.

An alternative embodiment of a fusion cage 520 is shown in Figures 24. Fusion cage 520 includes a distal end 524 and an a proximal end 526. A thread 540 is defined on the external surface of the fusion cage 520. Defined by the thread 540 are flutes 542, 544, 546, which in this embodiment extend from the distal end 524 toward the proximal end 526. These flutes provide thread relief 552 allowing the fusion cage 520 to be self-tapping

Still an alternative and preferred embodiment as mentioned above is shown in Figure 25. In this embodiment the fusion cage 620 includes a blunt or flat distal end 624 and a proximal end 626. As in the other embodiments, the fusion cage is conically-shaped, and includes a thread 640 and aperture 654.

Figure 26 includes a view of the distal end 624 of the fusion cage 620. This distal end 624 uses a snub nosed portion that is closed. The diameter of the snub nosed portion 660 is smaller than the largest root of the thread 640 at the distal end 624. As can be seen in Figure 26, the thread 640 has a starter portion or starter turn 641 which includes approximately the first half turn of the thread 640. The diameter of the starter portion 641, as can be seen Figure 26, is substantially less than the outside diameter of the four turns of thread 640 which comprised the cylindrical portion 625. From the cylindrical portion 625, the cage 620 and the thread 640 taper off to the proximal end 626 and define the conically-shaped portion 623.

The starter turn 641 of thread 640, as the name implies, assist in promoting the proper engagement of the thread 640 with the upper and lower vertebral bodies. In this embodiment, as in prior embodiments, the distal end has a diameter of approximately 16 millimeters. The diameter of the snub nosed portion 660 is about 10 millimeters.

### Industrial Applicability

The posterior fusion cage can be introduced through a posterior approach in order to maintain or increase lordosis between adjacent vertebral bodies. The fusion cage has the advantage of being conically-shaped and self-tapping through the use of external flutes. The flutes additionally assist in keeping the fusion cage aligned and centered as the cage is being inserted between the vertebral bone structures.

Other advantages, aspects, and objects of the invention can be obtained through a review of the claims.

## Claims

1. A fusion cage (20) for promoting fusion with one of more bone structures comprising:
a cage body (22) having a proximal end (26) and a distal end (24) said proximal end (26) having a diameter which is larger than a diameter of said distal end (24);
said proximal and distal ends (24, 26) define the opposite ends of a frusto-cone
**characterised in that** a conical thread (40) is formed into the outer surface (41) of the cage body (22).

2. The fusion cage of claim 1 wherein:
said cage body has at least one flute (42, 44, 46) in the thread (40).

3. The fusion cage of claim 2, wherein the flute (42, 44, 46) is formed in the distal end (24) in order to facilitate the insertion of the fusion cage (20) in the one or more bone structures, the flute (42, 44, 46) extending from the distal end toward the proximal end.

4. The fusion cage of claim 2 or 3 including:
at least three flutes (42, 44, 46) formed in the outer surface being equally spaced about said distal end (24).

5. The fusion cage of any of claims 2 to 4 wherein the thread (40) has a plurality of turns found on the outer surface, and the flute (42, 44, 46) is formed in at least one of said turns.

6. The fusion cage of any of preceding claims wherein:
said cage body (22) has an internal cavity (30); and
a plurality of apertures (54) are formed through the cage body (22) which communicate said outer surface with said internal cavity (40).

7. The fusion cage of claim 6 wherein said apertures (54) increase in size from the distal end (24) toward the proximal end (26).

8. The fusion cage of an of the preceding claims wherein said cage body (22) is a frustum of a right circular cone.

9. The fusion cage of any one of the preceding claims wherein:
said cage body (22) has a longitudinal axis, and a first transverse axis which is perpendicular to the longitudinal axis (50) and a second transverse axis which is perpendicular to both the longitudinal axis (50) and the first transverse axis; and has a position indicator (36, 38) located at said proximal end (26), which position indicator is located along the first transverse axis; and
said cage body (22) has a plurality of apertures (54) for promoting bone growth into the cage body (22), which apertures (54) are located only substantially along the second transverse axis between the proximal end (26) and the distal end (24).

10. The fusion cage of claim 9 wherein:
said position indicator includes an indentation (36, 38) into said proximal end (26).

11. The fusion cage of any one of the preceding claims wherein:
said distal end (24) has a snub nose extending therefrom in order to facilitate insertion relative to one or more bone structures, the snub nose having a diameter which is less than the diameter of the distal end (24).

12. The fusion cage of claim 11 wherein:
the cage body (22) includes a thread starter turn located at the distal end (24), the starter turn of the thread (40) extending from the snub nose in order to facilitate insertion relative to the one or more bone structures.

13. The fusion cage of any of the preceding claims in combination with an insertion tool (62), said fusion cage (20) including:
said proximal end (26) having an opening (28) which communicates with an internal cavity (30);
an end cap (32) which can fit into said opening (28) in order to close off said internal cavity (30);
said proximal end (26) including at least one insertion-tool-receiving indentation (36, 38);
said end cap (32) including an insertion-tool-receiving threaded bore (34) with an unthreaded portion with an irregularity; and
said insertion tool (62) including:
a tang (78, 80) for being received in said indentation (36, 38) and a threaded shaft (74) for being received in said threaded bore (34), and a shaft for mating with the unthreaded portion, said insertion tool (62) for being engaged with said fusion cage (20) for inserting said fusion cage (20) relative to the one or more bone structures.

14. A fusion cage of an one of claims 1 to 12, in combination with an insertion tool (700) and comprising:
at least one insertion-tool-receiving indentation (636, 638) in the proximal end (426) of the cage body (422);
said proximal end (426) including an insertion-tool-receiving threaded bore having an unthreaded portion with at least one irregularity; and
said insertion tool (700) having a first shaft (712) with a tang (714, 716) that can be received in said indentation (636, 638), a second shaft (710) with a portion which can mate with the unthreaded portion of the bore with the irregularity, and a third shaft (726) with a threaded portion which can mate with the threaded bore.

15. The fusion cage and insertion tool combination of claim 14 wherein:
said second and said third shafts (710, 726) can rotate relative to the first shaft (712) and relative to each other.

16. The fusion cage and insertion tool combination of claim 15 wherein:
one of said second and third shafts (710, 726) is biased relative to the other of said second and third shafts (710, 726).

17. The fusion cage and insertion tool combination of claim 16 wherein:
said fusion cage (720) includes an end cap (32) which in part comprises said proximal end, and wherein said end cap (32) includes said threaded bore (34), and wherein said end cap (32) can be selectively removed from the remainder of the proximal end.

## Patentansprüche

1. Fusionskäfig (20) zum Steigern der Fusion mit einer oder mehreren Knochenstrukturen, umfassend:
einen Käfigkörper (22) mit einem proximalen Ende (26) und einem distalen Ende (24), wobei das proximale Ende (26) einen Durchmesser aufweist, der größer ist als ein Durchmesser des distalen Endes (24);
das proximale und distale Ende (24, 26) die gegenüberliegenden Enden eines Kegelstumpfes bestimmen,
**dadurch gekennzeichnet, dass** ein konisches Gewinde (40) in der äußeren Oberfläche (41) des Käfigkörpers (22) gebildet ist.

2. Der Fusionskäfig nach Anspruch 1, wobei der Käfigkörper mindestens eine Rille (42, 44, 46) in dem Gewinde (40) aufweist.

3. Der Fusionskäfig nach Anspruch 2, wobei die Rille (42, 44, 46) in dem distalen Ende (24) gebildet ist, um die Einführung des Fusionskäfigs (20) in die eine oder die mehreren Knochenstrukturen zu erleichtern, die Rille (42, 44, 46) sich von dem distalen Ende zum proximalen Ende hin erstreckt.

4. Der Fusionskäfig nach Anspruch 2 oder 3, umfassend mindestens drei Rillen (42, 44, 46), die in der äußeren Oberfläche gebildet sind und die gleichmäßig zueinander um das distale Ende (24) beabstandet sind.

5. Der Fusionskäfig nach einem der Ansprüche 2 bis 4, wobei das Gewinde (40) eine Mehrzahl von Windungen auf der äußeren Oberfläche aufweist, und die Rille (42, 44, 46) in mindestens einer der Windungen gebildet ist.

6. Der Fusionskäfig nach einem der vorhergehenden Ansprüche, wobei
der Käfigkörper (22) einen Innenhohlraum (30) aufweist; und
eine Mehrzahl von Öffnungen (54) durch den Käfigkörper (22) gebildet sind, die die äußere Oberfläche mit dem Innenhohlraum (40) verbinden.

7. Der Fusionskäfig nach Anspruch 6, wobei die Größe der Öffnungen (54) von dem distalen Ende (24) zu dem proximalen Ende (26) hin zunehmen.

8. Der Fusionskäfig nach einem der vorhergehenden Ansprüche, wobei der Käfigkörper (22) ein gerader Kreiskegelstumpf ist.

9. Der Fusionskäfig nach einem der vorhergehenden Ansprüche, wobei
der Käfigkörper (22) eine longitudinale Achse, und eine erste transversale Achse, die senkrecht zur longitudinalen Achse (50) ist, und eine zweite transversale Achse, die senkrecht sowohl zur longitudinalen Achse (50) und zur ersten transversalen Achse ist, aufweist; und einen Positionsindikator (36, 38) aufweist, der sich an dem proximalen Ende (26) befindet, und der Positionsindikator sich entlang der ersten transversalen Achse befindet; und
der Käfigkörper (22) eine Mehrzahl von Öffnungen (54) zum Steigern des Knochenwachstums in den Käfigkörper (22) aufweist, die Öffnungen (54) nur im Wesentlichen entlang der zweiten transversalen Achse zwischen dem proximalen Ende (26) und dem distalen Ende (54) sich befinden.

10. Der Fusionskäfig nach Anspruch 9, wobei
der Positionsindikator eine Aussparung (36, 38) in dem proximalen Ende (26) umfasst.

11. Der Fusionskäfig nach einem der vorhergehenden Ansprüche, wobei
das distale Ende (24) eine Stupsnase aufweist, die sich von diesem erstreckt, um die Einführung relativ zu der einen oder den mehreren Knochenstrukturen zu erleichtern, und wobei die Stupsnase einen Durchmesser besitzt, der geringer ist als der Durchmesser des distalen Endes (24).

12. Der Fusionskäfig nach Anspruch 11, wobei
der Käfigkörper (22) einen Gewindeanfangswindung umfasst, der sich an dem distalen Ende (24) befindet, die Anfangswindung des Gewindes (40) sich von der Stupsnase erstreckt, um die Einführung relativ zu der einen oder den mehreren Knochenstrukturen zu erleichtern.

13. Der Fusionskäfig nach einem der vorhergehenden Ansprüche in Kombination mit einem Einführwerkzeug (62), wobei der Fusionskäfig (20) umfasst:
das proximale Ende (26) weist eine Öffnung (28) auf, die mit einem Innenhohlraum (30) in Verbindung steht;
eine Endabdeckung (32), die in die Öffnung (28) eingepasst werden kann, um den Innenhohlraum (30) abzuschließen;
das proximale Ende (26) mindestens eine Aussparung (36, 38) zum Aufnehmen eines Einführwerkzeuges umfasst;
die Endabdeckung eine Gewindebohrung (34) zum Aufnehmen eines Einführwerkzeuges mit einem Abschnitt ohne Gewinde mit einer Irregularität umfasst; und wobei
das Einführwerkzeug (62) umfasst:
einen Backen (78, 80), der in der Aussparung (36, 38) aufgenommen wird, und einen Gewindeschaft (74), der in der Gewindebohrung (34) aufgenommen wird, und einen Schaft, der das Gegenstück zu dem Abschnitt ohne Gewinde bildet, das Einführwerkzeug (62) mit dem Fusionskäfig (20) in Eingriff gebracht wird zum Einführen des Fusionskäfigs (20) relativ zu der einen oder den mehreren Knochenstrukturen.

14. Ein Fusionskäfig nach einem der Ansprüche 1 bis 12, in Kombination mit einem Einführwerkzeug (700) und umfassend:
mindestens eine Aussparung (636, 638) zum Aufnehmen eines Einführwerkzeuges in dem proximalen Ende (426) des Käfigkörpers (422) ;
das proximale Ende (426) eine Gewindebohrung zum Aufnehmen eines Einführwerkzeuges mit einem Abschnitt ohne Gewinde mit mindestens einer Irregularität umfasst; und
das Einführwerkzeug (700) einen ersten Schaft (712) mit einer Backe (714, 716), die in der Aussparung (636, 638) aufgenommen werden kann, und einen zweiten Schaft (710) mit einem Abschnitt, der das Gegenstück zu dem Abschnitt ohne Gewinde der Bohrung mit der Irregularität bildet, und einen dritten Schaft (726) mit einem Gewindeabschnitt, der das Gegenstück zur Gewindebohrung bildet, umfasst.

15. Die Kombination aus Fusionskäfig und Einführwerkzeug nach Anspruch 14, wobei
der zweite und der dritte Schaft (710, 726) sich relativ zu dem ersten Schaft (712) und relativ zueinander drehen können.

16. Die Kombination aus Fusionskäfig und Einführwerkzeug nach Anspruch 15, wobei
einer der beiden zweiten und dritten Schäfte (710, 726) relativ zu dem anderen der beiden zweiten und dritten Schäfte (710, 726) vorgespannt ist.

17. Die Kombination aus Fusionskäfig und Einführwerkzeug nach Anspruch 16, wobei
der Fusionskäfig (720) eine Endabdeckung (32) umfasst, die teilweise das proximale Ende aufweist, und wobei die Endabdeckung (32) die Gewindebohrung (34) aufweist, und wobei die Endabdeckung (32) wahlweise von dem verbliebenen Bereich des proximalen Endes entfernt werden kann.

## Revendications

1. Cage de fusion (20) pour encourager la fusion avec une ou plusieurs structures osseuses, comprenant :
- un corps de cage (22) ayant une extrémité proximale (26) et une extrémité distale (24), ladite extrémité proximale (26) ayant un diamètre qui est plus grand qu'un diamètre de ladite extrémité distale (24) ;
- lesdites extrémités proximale et distale (24, 26) définissent les extrémités opposées d'un cône tronqué,
**caractérisée en ce qu'**un filet conique (40) est formé dans la surface extérieure (41) du corps de cage (22).

2. Cage de fusion selon la revendication 1, dans laquelle ledit corps de cage présente au moins une rainure (42, 44, 46) dans le filet (40).

3. Cage de fusion selon la revendication 2, dans laquelle la rainure (42, 44, 46) est formée dans l'extrémité distale (24) pour faciliter l'insertion de la cage de fusion (20) dans une ou plusieurs structures osseuses, la rainure (42, 44, 46) s'étendant de l'extrémité distale vers l'extrémité proximale.

4. Cage de fusion selon la revendication 2 ou 3, incluant au moins trois rainures (42, 44, 46) formées dans la surface extérieure qui sont espacées uniformément autour de ladite extrémité distale (24).

5. Cage de fusion selon l'une quelconque des revendications 2 à 4, dans laquelle le filet (40) présente plusieurs tours se trouvant sur la surface externe, et la rainure (42, 44, 46) est formée dans au moins l'un desdits tours.

6. Cage de fusion selon l'une quelconque des revendications précédentes, dans laquelle ledit corps de cage (22) possède une cavité interne (30) ; et plusieurs ouvertures (54) sont ménagées dans le corps de cage (22) qui font communiquer ladite surface extérieure avec ladite cavité interne (40).

7. Cage de fusion selon la revendication 6, où lesdites ouvertures (54) augmentent en taille de l'extrémité distale (24) vers l'extrémité proximale (26).

8. Cage de fusion selon l'une quelconque des revendications précédentes, dans laquelle ledit corps de cage (22) est le tronc d'un cône circulaire droit.

9. Cage de fusion selon l'une des revendications précédentes, dans laquelle ledit corps de cage (22) possède un axe longitudinal, et un premier axe transversal qui est perpendiculaire à l'axe longitudinal (50) et un second axe transversal qui est perpendiculaire à la fois à l'axe longitudinal (50) et au premier axe transversal ; et qui comporte un indicateur de position (36, 38) situé à ladite extrémité proximale (26), cet indicateur de position se situant le long du premier axe transversal ; et ledit corps de cage (22) possède une pluralité d'ouvertures (54) pour encourager la croissance de l'os dans le corps de cage (22), ces ouvertures (54) étant situées seulement sensiblement le long du second axe transversal entre l'extrémité proximale (26) et l'extrémité distale (24).

10. Cage de fusion selon la revendication 9, dans laquelle ledit indicateur de position comprend une encoche (36, 38) dans ladite extrémité proximale (26).

11. Cage de fusion selon l'une des revendications précédentes, dans laquelle ladite extrémité distale (24) présente un nez d'amortissement s'étendant à partir de celle-ci pour faciliter l'insertion relativement à une ou plusieurs structures osseuses, le nez d'amortissement ayant un diamètre qui est plus petit que le diamètre de l'extrémité distale (24).

12. Cage de fusion selon la revendication 11, dans laquelle le corps de cage (22) comprend un tour de démarrage de filet situé à l'extrémité distale (24), le tour de démarrage du filet (40) s'étendant à partir du nez d'amortissement pour faciliter l'insertion relativement à une ou plusieurs structures osseuses.

13. Cage de fusion selon l'une quelconque des revendications précédentes en combinaison avec un outil d'insertion (62), ladite cage de fusion (20) incluant :
- ladite extrémité proximale (26) ayant une ouverture (28) qui communique avec une cavité interne (30) ;
- un capuchon d'extrémité (32) qui peut s'adapter dans ladite ouverture (28) pour fermer ladite cavité interne (30) ;
- ladite extrémité proximale (26) incluant au moins une encoche de réception d'outil d'insertion (36, 38) ;
- ledit capuchon d'extrémité (32) incluant un perçage fileté de réception d'outil d'insertion (34) avec une portion non filetée avec une irrégularité ; et
ledit outil d'insertion (62) incluant :
- une queue (78, 80) destinée à être reçue dans ladite encoche (36, 38) et une tige filetée (74) destinée à être reçue dans ledit perçage fileté (34), et une tige pour s'adapter à la portion non filetée, ledit outil s'insertion (62) est destiné à être mis en prise avec ladite cage de fusion (20) pour insérer ladite cage de fusion (20) relativement à une ou plusieurs structures osseuses.

14. Cage de fusion selon l'une des revendications 1 à 12, en combinaison avec un outil d'insertion (700) et comprenant :
- au moins une encoche de réception d'outil d'insertion (636, 638) dans l'extrémité proximale (426) du corps de cage (422) ;
- ladite extrémité proximale (426) incluant un perçage fileté de réception d'outil d'insertion possédant une portion non filetée avec au moins une irrégularité ; et
- ledit outil d'insertion (700) ayant une première tige (712) avec une queue (714, 716) qui peut être reçue dans ladite encoche (636, 638), une deuxième tige (710) avec une portion apte à s'adapter à la portion non filetée du perçage présentant l'irrégularité, et une troisième tige (726) avec une portion filetée qui peut s'adapter au perçage fileté.

15. Combinaison de cage de fusion et d'outil d'insertion selon la revendication 14, dans laquelle lesdites deuxième et troisième tiges (710, 726) peuvent tourner relativement à la première tige (712) et l'une relativement à l'autre.

16. Combinaison de cage de fusion et d'outil d'insertion selon la revendication 15, dans laquelle l'une des deuxième et troisième tiges (710, 726) est sollicitée relativement à l'autre desdites deuxième et troisième tiges (710, 726).

17. Combinaison de cage de fusion et d'outil d'insertion selon la revendication 16, dans laquelle ladite cage de fusion (720) comprend un capuchon d'extrémité (32) qui comprend en partie ladite extrémité proximale, et où ledit capuchon d'extrémité (32) comprend ledit perçage fileté (34), et où ledit capuchon d'extrémité (32) peut être retiré sélectivement du restant de l'extrémité proximale.
